# EUROPEAN PATENT APPLICATION

(11) **EP 1 435 242 A1**
(43) Date of publication of application: **07.07.2004**
(21) Application number: 02029107.6
(22) Date of filing: 31.12.2002
(51) Int. Cl.: A61K 35/78, A61P 7/00

(54) **Method of improvement of blood circulation**

(71) Applicant: Pharmaton S.A., 6934 Bioggio (CH)
(72) Inventor: The designation of the inventor has not yet been filed
(74) Representative: Kompter, Michael, Dr.

(57) **Abstract**

The invention relates to a method for the enhancement of the blood circulation and/or the oxygen supply of the lower extremities, which method comprises administering an effective amount of a pharmaceutical or dietary composition containing an aqueous extract of red vine leaves to the a person in need thereof

## Description

### BACKGROUND OF THE INVENTION

### 1. TECHNICAL FIELD

The invention relates to a method for the enhancement of the blood circulation and/or the oxygen supply of the lower extremities.

### 2. BACKGROUND INFORMATION

Chronic venous insufficiency (CVI) is a progredient disease and will lead in many patients - especially if untreated - to oedema, coronal phlebectasia (Widmer stage I), hyperpigmentation, induration, lipodermatosclerosis, white atrophy (Widmer stage II), or varicose leg ulcers (Widmer stage III). Chronically disturbed haemodynamics of deep or superficial veins due to obstructed venous segments or valvular incompetence lead usually to skin diseases in the inner ankle area of the lower limbs.1 Disturbances in the microcirculation of the skin have been considered to be major contributors for skin changes associated with chronic venous hypervolaemia and venous hypertension. (e.g. Fagrell B Vital microscopy and the pathophysiology of deep venous insufficiency. Int Angiol 1995;14:18-22.; Jünger M, Klyscz T, Hahn M, Rassner G. Disturbed blood flow regulation in venous leg ulcers. Int J Microcirc 1996;16:259-265).

Obviously, cutaneous microangiopathy of clinical relevance such as enlarged, tortuous capillaries surrounded by micro-oedema contributes to the skin alterations in the lower limbs and determines the course of CVI (Fagrell B, *loc*. *cit.* and Jünger M et al., *loc. cit.*).

The application of the laser Doppler technique in venous disorders is well illustrated. (e.g. Tulevski II, Ubbink DT, Jacobs MJHM. Red and green laser Doppler compared with capillary microscopy to assess skin microcirculation in the feet of healthy subjects.

Microvasc Res 1999;58(2):83-88 and Bollinger A, Jäger K, Jünger M, Seifert H. The vascular laboratory: advances in non-invasive techniques. World J Surg 1988;12:724-731).

Different techniques have been developed to investigate microcirculation in both functionally different layers of the skin: the deeper, mainly thermoregulatory layer and the superficial, nutritive layer. Microcirculatory disturbances in the superficial nutritive layer are of utmost relevance for trophical skin changes. (Jünger M et al., *loc. cit.* and Gschwandtner ME, Ambrozy E, Fasching S, Willfort A, Schneider B, Böhler K, et al. Microcirculation in venous ulcers and surrounding skin: findings with capillary microscopy and laser Doppler imager. Eur J Clin Invest 1999;29:708-716).

The British patent GB 934,554 discloses that the capillary resistance of guniea pigs deficient in a vitamin can be enhanced by intraperitonally administration of an alcoholic extract of vine leaves.

The International patent application WO 01/28363 discloses a method for preventing or alleviating the discomfort associated with mild-to-moderate chronic venous insufficiency of the lower extremities with the aid of an aqueous extract of red vine leaves.

### BRIEF SUMMARY OF THE INVENTION

In a clinical trial it has been surprisingly found that the microcirculation and the oxygen supply at the predominantly affected perimalleolar area of the leg in CVI patients can be significantly improved by oral administration with an aqueous extract of red vine leaves.

Accordingly the invention relates to a method for the enhancement of the blood circulation and/or the oxygen supply of the lower extremities, which method comprises administering an effective amount of a pharmaceutical or dietary composition containing an aqueous extract of red vine leaves to the a person in need thereof.

A further aspect of the present invention is a method for prevention of skin changes including prevention of blood clots in the veins or inflammatory reactions in small vessels associated with chronic venous insufficiency, chronic venous hypervolaemia and/or venous hypertension of the lower extremities, which method comprises administering an effective amount of a pharmaceutical or dietary composition containing an aqueous extract of red vine leaves to the a person in need thereof.

Furthermore, the invention relates to a method for the prevention or delay of the transition from clinically not relevant early stages of chronic venous insufficiency (CVI) to CVI Stage I, II, or III, which method comprises administering an effective amount of a pharmaceutical or dietary composition containing an aqueous extract of red vine leaves to the a person in need thereof.

### BRIEF DESCRIPTION OF THE DRAWINGS

Figure 1 shows the schematic design of the clinical study carried out.
Figure 2 shows the influence of the vine leaf extract
   - ―●―: AS 195 360 mg compared with
   - ―○―: placebo
   on the microcirculation measured with Laser Doppler flowmetry (LDF 10-37 kHz).
Figure 3 shows the influence of the vine leaf extract
   - ―●―: AS 195 360 mg compared with
   - ―○―: placebo
   on Transcutaneous oxygen partial pressure (tpO₂)

### DETAILED DESCRIPTION OF THE INVENTION

The composition of the present invention preferably consists of herbal ingredients derived by an aqueous extraction from red vine leaves (*folia vitis viniferae;* Extractum Vitis viniferae e folium spissum et siccum) and an acceptable carrier. This extract contains flavon(ol)-glycosides, -glucuronides and flavonoids, with quercetin-3-O-β-D-glucuronide and isoquercitrin (quercetin-3-O-β-glucoside) as its main active ingredients. The range of their pharmacological actions has not yet been fully elucidated, but in-vitro studies indicate that they have antioxidant and anti-inflammatory properties and that they inhibit platelet aggregation and hyaluronidase and reduce oedema, possibly by reducing capillary permeability. Preclinical in-vivo experiments demonstrated anti-inflammatory and capillary wall thickening effects.

In a preferred embodiment, the composition is in a form suitable for oral administration, in particular in a solid dosage form, i.e. a capsule or tablet, that consists of 20 to 60% of aqueous red vine leaf extract with a high flavonoid content of 2-15%. Another preferred dosage form is that of drops containing 3 to 90% of extract. Further suitable administration forms may be coated tablets, syrups, or the like. Most preferred are capsules and film coated tablets.

With the foregoing in mind, it is a primary object of the present invention to provide a composition for preventing and alleviating the discomfort associated with mild-to-moderate chronic venous insufficiency of the lower extremities.

It is a further object of the present invention to provide a composition for preventing and/or alleviating the discomfort associated with mild-to-moderate chronic venous insufficiency of the lower extremities comprising herbal ingredients, wherein the composition is manufactured pursuant to a controlled process that preserves the herbal curing qualities of the ingredients.

It is still a further object of the present invention to provide a composition which is effective in preventing and/or alleviating the discomfort associated with mild-to-moderate chronic venous insufficiency of the lower extremities.

It is still a further object of the present invention to provide a composition for preventing and/or alleviating the discomfort associated with mild-to-moderate chronic venous insufficiency of the lower extremities comprising herbal ingredients and having minimal or no side effects and thus being safe for internal consumption.

A fundamental part of the present invention is the preparation of a composition for oral administration containing an aqueous extract prepared from dried red vine leaves. The latter is characterised by a high content of 2 to 20%, preferably 2 to 10 % of biologically active flavonoids.

The term "a person in need thereof" relates hereinabove and hereinbelow to a person who suffers from clinically not relevant early stages of chronic venous insufficiency (CVI) or has proven CVI stage I and II according to Widmer. As a rule such patients are elderly people with an age of between 30 and 80, preferably between 32 and 76 years having an mean age (± standard deviation) of 55.2 ± 7.7 years.

In order that this invention be more fully understood, the following examples are set forth. These examples are for the purpose of illustrating embodiments of this invention, and are not to be construed as limiting the scope of the invention in any way.

The examples which follow are illustrative and, as recognised by one skilled in the art, particular conditions could be modified as needed for individual compositions. Materials used in tests below are either commercially available or easily prepared from commercially available materials by those skilled in the art.

The basis of the composition is the aqueous extract of red vine leaves (*foliae vitis viniferae L.*). The starting material for the preparation of the extract are red vine leaves collected at a point of time where the content in flavonoids has reached an optimum. This is usually the case around the harvesting time of the grapes. The leaves are carefully dried and crushed. For extraction the leaves are cut to pieces of preferably 5 to 10 mm. To achieve a high content in flavonoids the extraction is done at elevated temperature, preferably at a temperature in the range of 60° to 80°C, over a time of at least 6 up to 10 hours. The preferred method is that of an exhaustive percolation.

The so-called fluid extract obtained in the course of the extraction may be directly used in the preparation of liquid dosage forms. In order to get a more concentrated extract preferably at least part of the solvent is removed by use of a suitable evaporator. The thick extract obtained in this step may again be directly used in the manufacturing of liquid dosage forms.

For the preparation of solid dosage forms the thick extract is dried, for instance by use of a vacuum drying oven or a vacuum drying conveyer. Carriers or excipients may be added during drying to facilitate further processing of the extract. Such carriers or excipients may be silicon dioxide, maltodextrine, glucose syrup, cellulose and others.

The composition for oral administration is manufactured using usual techniques applied in the food industry or in the pharmaceutical industry. Preferred administration forms are tablets, including coated tablets or capsules. But also liquid preparations, preferably drops, may be chosen.

Most preferred is a film coated tablet containing 300 to 500 mg, preferably 320 to 400 mg, in particular about 360 mg of dry aqueous extract of red vine leaf (4-6 : 1). (*extractum vitis viniferae foliae aquosum siccum*) and the following excipients:
hypromellose, glyceryl tristearate, titanium dioxide (E 171), talc, ferric oxide, red (E 172), microcrystalline cellulose, croscarmellose sodium, calcium hydrogen phosphate (anhydrous), colloidal silica (anhydrous), magnesium stearate.

These film coated tablets are hereinbelow coded "AS 195".

To enhance the blood circulation and/or the oxygen supply of the lower extremities, the composition should be taken in dosages corresponding to 80 and 1000 mg of extract, preferably 300-800 mg, in particular 350-750 mg daily. The total amount of extract may be divided up in 1 to 3 capsules or tablets a day (or an equivalent dose by means of a liquid form). The daily dose should be taken at once, preferably in the morning.

Impressive improvement of the symptoms can be expected within 6 weeks of continuous use. The optimum effect is maintained or amplified on longer use.

### Methods

### Participants

Male and female patients, age 18 years or more, with proven CVI I or CVI according to Widmer, with diagnosis confirmed and present for at least one year were enrolled. Medically relevant concomitant diseases have to be absent. Patients who used drugs to alleviate their CVI symptoms within 4 weeks or were treated with theophyllin, diuretics, cardiac glycosides, ACE inhibitors or calcium antagonists within 8 days prior to the first examination were not allowed to be enrolled. Compression bandages or concomitant therapy for venous problems were forbidden during the participation in the trial.

### Design and procedures

The double-blind, randomised, placebo-controlled cross-over trial was run according to the principles of the declaration of Helsinki and the International Conference of Harmonisation of Good Clinical Practice.

Each patient participated for 17 weeks in the trial: for a one-week wash-out (placebo-treated), for a 6-week treatment period (Group_1 starting with AS 195, Group_2 starting with placebo), for a 4-week wash-out (placebo-treated), and for a second 6-week treatment period (Group_1 continuing with placebo, Group_2 continuing with the ).

AS 195 (film-coated tablets containing 360 mg dry extract of red vine leaves) or placebo tablets were taken according to the randomisation schedule as single dose in the morning. Both tablets were identical with respect to size, shape, weight, inner appearance, and taste. For laser Doppler flowmetry the equipment was provided by LMTB, Berlin, Germany (e.g. Doerschel K, Mueller G. Velocity resolved laser Doppler flow measurement in skin. Lasermedizin 1996;12:163-171.). The equipment is a computer-based mobile unit using a laser frequency of 785 nm. The laser probe was fixed 3.5 cm distal to the inner ankle of the more affected leg. After 30 minutes sitting for adaptation to room temperature, measurement started after 10 minutes standing (256 points of measurement, duration of measurement: approx. 0.4 seconds). The back-scattered light was retrieved by two diodes in the range of frequencies between 0.2 to 37.2 kHz. The data were processed using a Fast Fourier Transformation. Finally, the output referred to the range of frequencies between 0.2 to 10.0 kHz for vessels in the reticular venous plexus (larger mainly thermoregulative vessels, diameter more than 30 micrometer) and to the range of frequencies between 10.1 to 37.2 kHz for capillaries in the subpapillary venous plexus (superficial small nutritive vessels, diameter 6 to 30 micrometer).

Transcutaneous oxygen pressure (tcPO2) was measured using modified Clark-type polarographic electrodes containing noble metal cathodes and silver/sliver chloride anodes (TCM 3, Radiometer Copenhagen, Brønshøj, Denmark). A heating element adjacent to the anode maintained skin temperature at 43° Celsius. At this temperature the arterioles are maximally dilated, tcPO2 approximates the PO2 of arterial blood (e.g. Bollinger A, Jäger K, Jünger M, Seifert H. The vascular laboratory: advances in non-invasive techniques. World J Surg 1988;12:724-731.).

The electrode was attached to the skin surface by an adhesive ring device which was filled with physiological saline, 3.5 cm anteriolateral from the Laser Doppler probe. After 30 minutes sitting for adaptation to room temperature, measurement started after 10 minutes of standing. A measurement lasted approx. 15 minutes. The tcPO₂ values are expressed in millimeter mercury column (mmHg). Normal values available for the dorsum of the foot of patients without CVI are ranged between 40 and 80 mmHg.

Local skin temperature was measured with a thermistor fixed adjacent to the oxygen electrode in the perimalleolar region. In order to minimise effects on the skin perfusion, LDF and tcPO₂ measurements were conducted between 28 and 32° C local skin temperature.

Calf and ankle circumference were measured using a measuring tape. Measurements were carried out at the lateral and medial ankle and at the middle of the calf.

Subjective symptoms of CVI (tired heavy legs, sensation of tension, tingling sensation, and pain) were measured by using a 10-cm visual analogue scale with zero as "none at all" and 10 cm as "very strong".

Overall treatment efficacy was rated by patients and investigators on a 4-point verbal rating scale (good, satisfactory, not satisfactory, and bad) at the end of each treatment period.

Overall tolerability was rated by patients and investigators on a 4-point verbal rating scale (good, satisfactory; not satisfactory, and bad). The patients were questioned about their well-being in general terms at each visit.
Laboratory safety screens (haematology, clinical chemistry, and urinalysis) and general physical examinations were performed two times during the study. Blood pressure and heart rate while sitting were measured at each visit.

### Results

Seventy-one women and men aged between 32 and 76 years with proven CVI stage I and II according to Widmer were included. The mean age (± standard deviation) was 55.2 ± 7.7 years; 55 were women, 16 men. The phlebological status revealed moderate or severe intensity of varicosis in 47 (67.1%), pigmentation in 27 (38.6%), ankle oedema in 26 (37.1%), and lower leg oedema in 25 (35.7%) patients. Mild signs of atrophy were present in 13 patients (18.6%), of eczema in none (Table 1).

**Table 1:**

| **Demographics and baseline characteristics of CVI** | | |
|---|---|---|
| | **AS 195 / Placebo** **(n=36)** | **Placebo / AS195** **(n=35)** |
| **Continuous variates (median (range))** | | |
| Age [years] | 66 (32-76) | 66 (37-76) |
| Height [cm] | 168 (150-186) | 165 (150-191) |
| Weight [kg] | 76.5 (48-97) | 73 (55-120) |
| Body mass index [kg/m²] | 27.6 (20.6-32.0) | 26.7 (20.1-42.5) |
| Systolic blood pressure [mmHg] | 130 (100-150) | 135 (120-140) |
| Diastolic blood pressure [mmHg] | 80 (60-90) | 80 (65-90) |

| **Categorical variates (n (%))** | | |
|---|---|---|
| Female | 24(66.7) | 31 (88.6) |
| Current smoker | 4 (11.1) | 1(2.9) |
| CVI stage | | |
| Stage I | 26 (72.2) | 23 (65.7) |
| Stage II | 10(27.8) | 12(34.3) |
| Phlebological status of moderate | | |
| to severe intensity | | |
| Varicosis | 26 (72.2) | 22 (62.9) |
| Pigmentation | 11 (30.6) | 17 (48.6) |
| Atrophy | 0(0.0) | 0 (0.0) |
| Eczema | 0 (0.0) | 0 (0.0) |
| Ankle oedema | 13 (36.1) | 14 (40.0) |
| Lower leg oedema | 12 (33.3) | 14 (40.0) |

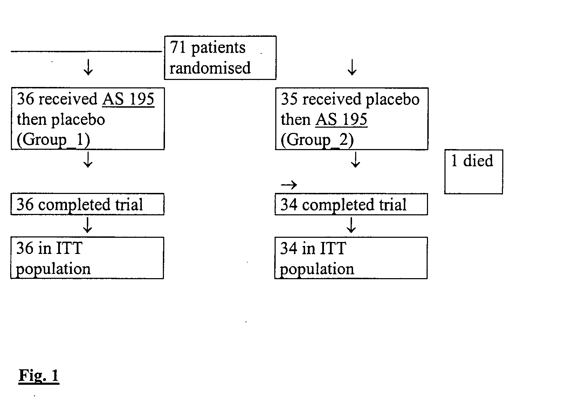

One 76-years old men died from a heart attack during a tennis match (while on placebo). This patient was excluded from the intention-to-treat analyses. Protocol violations did not occur in the remaining patients. Therefore, all 70 patients remained in the intention to treat analyses (Figure 1). Patient characteristics were homogenously distributed across the two treatment sequences (Group_1, Group_2) except for the sex ratio (12 men in Group_1, 4 men in Group_2) (Table 1). Baseline values for the laser Doppler parameters, transcutaneous oximetry, ankle and calf circumferences, and subjective symptoms were comparable for Group_1 and Group_2 (Table 2). Compliance was approximately 100% in both treatment sequences.

**Table 2:**

| **Mean (±SD) of baseline characteristics of each treatment period** | | | | |
|---|---|---|---|---|
| | **Period 1** | | **Period 2** | |
| | **AS 195** **(n=36)** | **Placebo** **(n=34)** | **AS 195** **(n=34)** | **Placebo** **(n=36)** |
| **Laser Doppler Flowmetry [AU]** | | | | |
| 10-37 kHz | 303.5 (135.2) | 333.5 (153.0) | 275.4 (126.4) | 293.3 (119.9) |
| < 10 kHz | 352.7 (87.7) | 370.8 (120.0) | 174.7 (77.0) | 189.4(67.6) |
| **Transcutaneous Oximetry [mmHg]** | 32.1 (7.0) | 32.3(6.4) | 30.1(6.2) | 30.8 (6.4) |

| **Circumference [cm]** | | | | |
|---|---|---|---|---|
| Ankle | 20.3(2.2) | 20.4(2.4) | 20.2 (2.6) | 20.3 (2.2) |
| Calf | 34.7 (3.1) | 34.2 (3.0) | 34.0 (3.1) | 34.6 (3.2) |

| **Subjective symptoms [cm]** | | | | |
|---|---|---|---|---|
| Tired/heavy legs | 4.3(2.8) | 3.7 (2.9) | 4.6 (2.9) | 5.2 (2.6) |
| Pain in legs | 4.0(3.2) | 3.2(3.1) | 4.5(2.7) | 4.9(3.1) |
| Sensation of tension | 4.5 (2.9) | 4.1 (2.8) | 4.5 (2.6) | 5.1(2.5) |
| Tingling sensation | 3.3 (3.1) | 2.7(2.9) | 3.7(2.6) | 4.2 (2.8) |

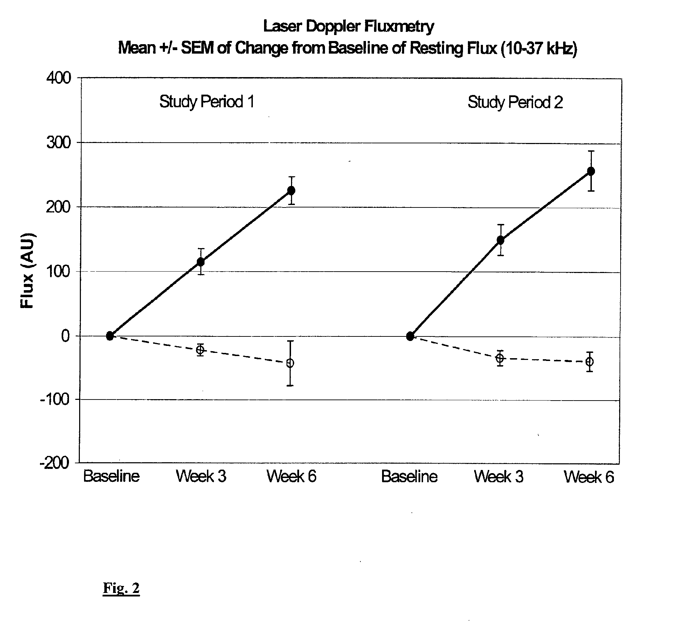

Laser Doppler Flow measurements in the frequency range of 10-37 kHz were elected for the primary endpoint. These frequencies are considered to be determined by the number of erythrocytes and their movements (flow velocity) in the capillaries of the superficial layer of the skin of the leg. After 6 weeks the laser Doppler frequencies (10-37 kHz) increased in the AS 195 group (plus 241.8±18.7 AU) but decreased in the placebo group (minus 41.0±18.7 AU, p<0.0001) (Table 3). This effect was present as early as 3 weeks after start of treatment (p<0.0001) (Table 4, Figure 2).

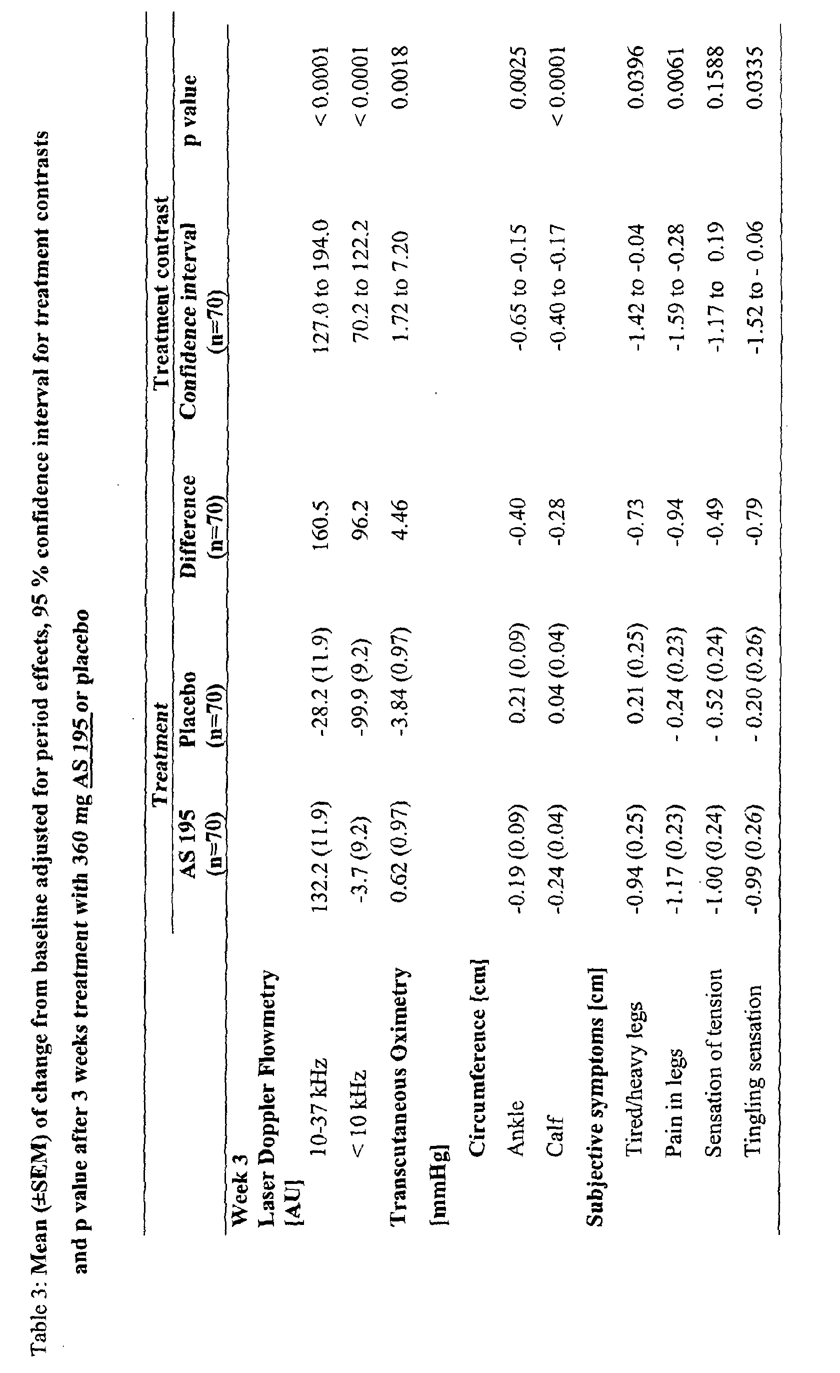

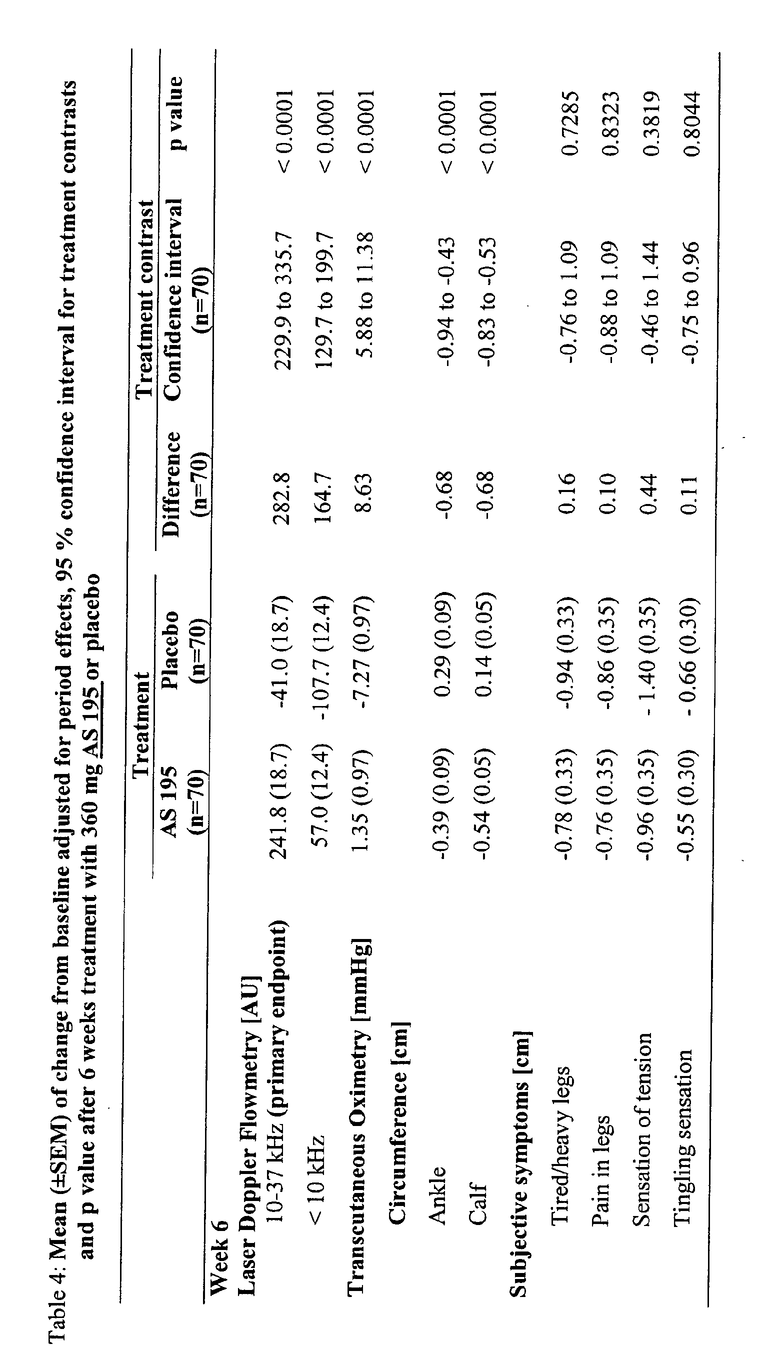

Laser Doppler Flow measurements in the frequency range below 10 kHz are considered to be determined by the number of erythrocytes and their movements (flow velocity) in the capillaries in the deeper mainly thermoregulative layer of the skin of the leg. After 6 weeks the laser Doppler frequencies below 10 kHz) increased in the AS 195 group (plus 57.0±12.4 AU) and decreased in the placebo group (minus 107.7±12.4 AU, p<0.0001) (Table 3). This effect seems to depend on the climatic condition during the treatment period. During the study period of moderate temperatures (April/May) the Laser Doppler measurements (<10 kHz) remained unchanged in the AS 195 treatment group after an initial drop whereas the measurements in the placebo group decreased (p<0.0001). During the study period of higher temperatures (July/August) the laser Doppler measurements (<10 kHz) increased in the AS 195 treatment group and remained constant in the placebo group. (p<0.0001).

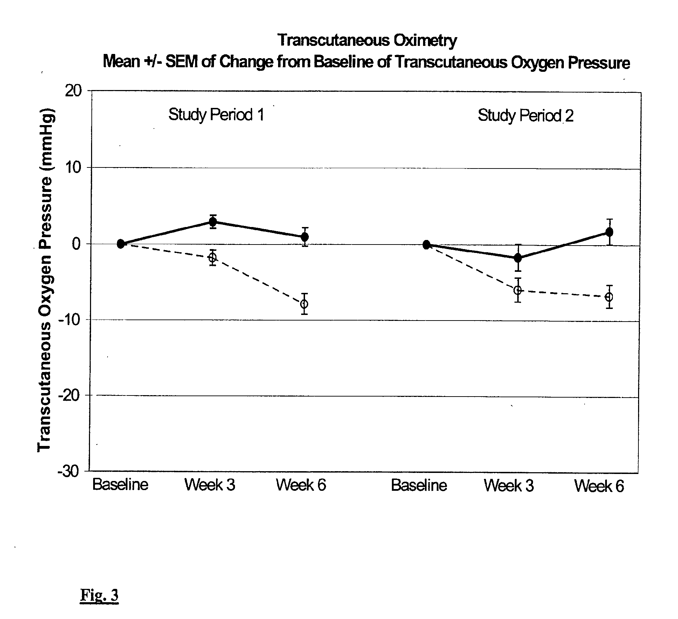

The transcutaneous oxygen pressure increased in the AS 195 group (plus 1.35±0.97 mmHg) but decreased in the placebo group (minus 7.27±0.97 mmHg, p<0.0001). This observation was consistent in both treatment periods and would therefore be in line with the Laser Doppler Flow in the nutritive superficial layer of the skin (i.e., 10-37 kHz) (Table 3,4, Figure 3).

The statistically significant and clinically relevant reduction of ankle (after 3 weeks: AS 195 minus 0.19±0.09 cm, placebo plus 0.21±0.09 cm, p=0.0025) and calf circumferences (after 3 weeks: AS 195 minus 0.24±0.04 cm, placebo plus 0.04±0.04 cm, p<0.0001) indicate an onset of action as early as 3 weeks after start of treatment (Table 3). This effect becomes more pronounced after 6 weeks (AS 195 ankle: minus 0.39±0.09 cm, calf: minus 0.54±0.05; placebo ankle: plus 0.29±0.09 cm, calf: plus 0.14±0.05 cm, p<0.0001) (Table 4)

There was no relevant change of the intensity of the subjective symptoms related to CVI after 6 weeks of treatment. This result is in line with those of a previous study where subjective symptoms measured on a visual analogue scale were reduced only after longer treatment periods (12 weeks).

Adverse events occurred rarely in this study. Thirteen of 71 patients experienced at least one adverse event, 12 of them experienced the onset of action while on placebo treatment, one while on AS 195 (bronchitis, moderate intensity, considered not drug related by the investigator). The patient who died from cardiac arrest had been treated with placebo (never received AS 195 in this trial). All patients assessed the overall tolerability as good or satisfactory. The laboratory parameters did not change during the study.

### Discussion

It has been shown in a previous study (WO 01/28363) that red vine leaves extract AS 195 reduces lower leg oedema, calf circumference, and ankle circumference in addition to improving subjective symptoms related to chronic venous insufficiency in patients treated once daily for 12 weeks.⁷ The present study was designed to provide additional information on the underlying mechanism of action by investigating microcirculation as a clinically relevant surrogate parameter for CVI related leg problems. This study is the first one in CVI patients aimed to investigate in addition to leg oedema reduction further clinical relevant effects related to the therapy with red vine leaves extract. The reduced venous drainage results in impaired cutaneous microcirculation with trophical disturbances of the skin. If CVI remains untreated this condition may even result venous leg ulcers. Laser Doppler flowmetry, as used in the present study, is a valid and sensitive method to measure objective treatment effects which may be related to the subjectively experienced volume reduction after 3 months of treatment.

The study results fit into the clinical data available for AS 195 and add information on the onset of action. The leg volume as an objective parameter will be reduced in a clinically relevant and statistically significant degree after 6 weeks of treatment. This objective effect has also been reported recently with horse chestnut sees extract (e.g. Diehm C, Trampisch HJ, Lange S, Schmidt C. Comparison of leg compression stocking and oral horse-chestnut seed extract therapy in patients with chronic venous insufficiency. Lancet 1996;347:292-294.) and Butchers Broom (e.g. Vanscheidt W, Jost V, Wolna P, et al. Efficacy and safety of a Butcher's Broom preparation (Ruscus aculeatus L. extract) compared to placebo in patients suffering from chronic venous insufficiency. Drug Res 2002;52(4):243-250.).

In the present study it was shown that the laser Doppler flowmetry parameters, the ankle and calf circumferences and the transcutaneous oxygen pressure were affected as early as after 3 weeks of treatment. In contrast, the subjective symptoms of CVI rated on a visual analogue scale were not significantly different from placebo after 6 weeks of treatment as they were in the previous study. A treatment duration of 12 weeks is mandatory for a relevant reduction of subjective CVI symptoms.

The present results suggest a major role of red vine leaves extract in prevention of CVI progression and the occurrence of trophical skin lesions and may even prevent or delay the transition from clinically not relevant early stages of CVI to CVI Stage I.

## Claims

1. A method for the enhancement of the blood microcirculation and/or the oxygen supply of the skin of the lower extremities, which method comprises administering an effective amount of a pharmaceutical or dietary composition containing an aqueous extract of red vine leaves to the a person in need thereof.

2. A method for prevention of skin changes including prevention of blood clots in the veins or inflammatory reactions in small vessels associated with chronic venous insufficiency, chronic venous hypervolaemia and/or venous hypertension of the lower extremities, which method comprises administering an effective amount of a pharmaceutical or dietary composition containing an aqueous extract of red vine leaves to the a person in need thereof.

3. A method for prevention or delay of the transition from clinically not relevant early stages of chronic venous insufficiency (CVI) to CVI Stage I, II or III, which method comprises administering an effective amount of a pharmaceutical or dietary composition containing an aqueous extract of red vine leaves to the a person in need thereof.

4. A method according to any one of claim 1 to 3, wherein the composition is in a form suitable for oral administration.

5. A method according to any one of claims 1 to 4 wherein said red vine leaf extract contains at least 2 and up to 20% flavonoids.

6. A method according to claim 5 wherein said red vine leaf extract contains at least 2 and up to 10% flavonoids.

7. A method according to any of the preceding claims wherein flavonoids are present within the range of 0.1% to 15% related to the total mass of the composition.

8. A method according to claim 7 wherein flavonoids are present within the range of 1% to 10% related to the total mass of the composition.

9. A method according to any of the preceding claims wherein said red vine leaf extract is present within the range of 1 to 90% related to the total mass of the composition.

10. A method according to claim 9 wherein said red vine leaf extract is present within the range of 1 to 70% related to the total mass of the composition.

11. A method according to claim 7 wherein said red vine leaf extract is present within the range of 1 to 50% related to the total mass of the composition.

12. A method according to any of the preceding claims, wherein the composition is in a form suitable of film tablets or capsules.

13. A method according to any of the preceding claims, wherein the composition is administered in dosages corresponding to 80 and 1000 mg of extract daily.

14. A method according to claim 13, wherein the composition is administered in dosages corresponding to 300-800 mg of extract daily

15. A method according to any of the preceding claims, wherein the total amount of extract is divided up in 1 to 3 film tablets a day.

16. A method according to any of the preceding claims, wherein the aqueous extract of red vine leaves is obtainable by a method comprising the steps of:
(a) collecting red vine leaves at a point of time when the content in flavonoids has reached an optimum;
(b) drying and crushing the leaves;
(c) cutting the leaves to pieces;
(d) extracting the leaves with water at elevated temperatures for 6 to 10 hours;
(e) optionally concentrating the obtained extract.

17. A method according to claim 16, wherein the leaves in step (d) are extracted with water at temperatures from 60 to 80 °C.
